# EUROPEAN PATENT SPECIFICATION

(11) **EP 2 777 724 B1**
(45) Date of publication and mention of the grant of the patent: **30.01.2019**
(21) Application number: 14159212.1
(22) Date of filing: 12.03.2014
(51) Int. Cl.: A61L 27/42, A61L 27/58, A61L 31/12, A61L 31/14, A61L 17/06

(54) **Composite material and uses thereof**
Verbundstoffmaterialien und Verwendungen davon
Matériau composite et ses utilisations

(30) Priority: 14.03.2013 US 201313829033
(43) Date of publication of application: 17.09.2014
(73) Proprietor: St. Jude Medical Coordination Center BVBA, 1930 Zaventem (BE)
(72) Inventor: Örnberg, Andreas, West Saint Paul, MN Minnesota 55118 (US); Norlin-Weissenrieder, Anna, 18146 Lidingö (SE); Weissenrieder, Jonas, 181 46 Lidingö (SE)
(74) Representative: Brann AB

(56) References cited:
- EP-A2- 2 198 898
- WO-A1-2012/113624
- US-A1- 2005 126 663

## Description

### TECHNICAL FIELD

The present disclosure relates generally to a magnesium based composite material for an implant, said composite material comprising a catalyst, and further to biologically degradable medical implants comprising said composite material.

### BACKGROUND

Temporary implants that are biologically degradable offer the advantage that they need not be removed in a second surgical intervention after serving their purpose, which makes them both convenient for the patient and more economical. Biodegradable polymers are commonly used for implantation purposes. This group of polymers often exhibit desirable properties in terms of degradation rate and biocompatibility, but they often lack the necessary mechanical stability, and it is known that metal-based biodegradable implants may offer more suitable mechanical properties than polymers. For example, magnesium (Mg) and magnesium based alloys have become interesting candidates in the field of biodegradable implant materials.

In general, magnesium and its alloys have great potential as biodegradable materials, since magnesium is an essential element in the human body. Mg degrades in aqueous solutions and offers a mechanically more stable construction of the implant than degradable polymeric materials (Hänzi et al., Acta Biomaterialia, 2009, 5:162-171). The entire contents of this publication is incorporated herein by reference for the Mg and Mg based materials and related processes described therein. Mg and its alloys have been used for biodegradable orthopaedic implants, and their application to coronary stents and other vascular devices is currently being investigated. It has been considered that the mechanical and corrosion properties of pure magnesium do not suit the requirements for stent material. However, magnesium alloys are seen as promising biodegradable implant materials (Mani et al., Biomaterials, 2007, 28: 1689-1710).

Magnesium and its alloys exhibit one major drawback when allowed to degrade in the body by its natural reaction. Degradation of magnesium in aqueous solutions at pH values below 11.5 (typically the pH within a body is less than 11.5) is associated with the formation of hydroxide ions and gaseous hydrogen, as described by the following electrochemical reaction:

Mg + 2H₂O → Mg²⁺ + 2OH⁻ + H₂

This mechanism is highly insensitive to the oxygen concentration. In vivo, hydrogen ions can cause harm to tissues as the pH value increases locally, and hydrogen gas formation can cause gas pockets to form around the implant and thereby cause damage to the surrounding tissue or create gas bubble formation in the blood, which is dangerous to the patient.

The conventional method of solving the problems associated with hydrogen release from magnesium implants consists of slowing down the degradation rate and hence reducing the release rate of hydrogen gas and hydroxide ions. This is commonly done by enhancing the corrosion resistance of the material by various methods, such as alloying, modifying their microstructure or their surface, including the application of conversion coatings (Staiger et al., Biomaterials, 2006, 27: 1728-1734; Guangling Song, Corrosion Science, 2007, 49: 1696-1701; Wang et al., Advanced Materials Research, 2008, 32: 207-210). WO2012113624 A1 describes an implant comprising a base from a biocorrodible magnesium alloy, which may contain particles of one or more of the elements Y, Zr, Mn, Sc, Fe, Ni, Co, W, Pt. EP2198898 A2 relates to an implant, which comprises magnesium or magnesium alloy, and which on at least a part of its surface comprises a layer of a hydrogen binding material, preferably palladium.

Coating the metallic surface with other materials to alter its surface characteristics without interfering with the bulk properties of the metal implant has been one rational approach to address the issue relating to the compatibility of metallic stents with tissue and blood. The coating can improve the surface properties significantly; surface energy can be reduced, surface texture can be smoothened, surface potential can be neutralised and the stability of the surface oxide layer can be enhanced. There are many inorganic coating materials which are potentially suitable for the treatment of medical implant surfaces. For example, gold and iridium oxide have been used as stent inorganic coating materials (Mani et al., Biomaterials, 2007, 28: 1689-1710), however not on magnesium-based implants.

None of the methods mentioned above reduce the total amount of hydrogen released during the degradation of the magnesium present in the implant. These methods only slow the release rate down as a result of the slower degradation rate.

Another method for addressing hydrogen evolution is the development of magnesium based metallic glasses. This material has extended solubility for alloying elements and a homogeneous single-phase structure which can lead to altered corrosion behavior and reduction of hydrogen evolution (Bruno Zberg, et al., Nature Materials, 2009, 8: 887-891). Often metallic glasses are difficult and expensive to manufacture in larger quantities as the material has to be cooled at a very rapid rate to obtain the amorphous structure. It makes it especially challenging to manufacture thick materials as the thicker it is, the more difficult it is to maintain an appropriate cooling rate within the bulk of the material. Another disadvantage with metallic glass is that it can be difficult to maintain its amorphous structure when it is subjected to processing steps, like laser cutting, annealing and shaping, that can induce crystallization of the material.

### SUMMARY

The present disclosure discloses the use of magnesium or magnesium based alloy, which is loaded with catalytic areas and/or surface treated with catalyst, which reduces the hydrogen gas evolution/release inside a person.

To successfully introduce magnesium and magnesium alloys into a clinical setting it is vital to reduce the total hydrogen released from the implant, not only its hydrogen release rate. The present disclosure solves or at least mitigates the problems described above, by providing implant materials having improved degradation properties, manifested by reduced hydrogen gas emission, compared to conventional magnesium based materials.

The present disclosure provides a material technology that may be used for a great variety of medical applications, such as coronary stents, clips and sutures, vascular closure devices and temporary attachment of pacemaker/neurostimulation means and orthopedic implants.

### BRIEF DESCRIPTION OF THE DRAWINGS

The accompanying drawings illustrate various embodiments of the present disclosure and are a part of the specification. The illustrated embodiments are merely examples of the present disclosure and do not limit the scope of the invention.
Fig. 1 is a schematic illustration of the catalytic conversion of hydrogen on degrading magnesium or magnesium alloy; and
Fig. 2 is a schematic illustration of a composite material according to one embodiment of the present invention.

### DETAILED DESCRIPTION

The present disclosure discloses the use of magnesium or magnesium based alloy, which is loaded with catalytic areas and/or surface treated with catalyst, which reduces the hydrogen gas evolution/release through one or several of the following mechanisms: reduction of the exchange current rate for hydrogen evolution, local and temporary adsorption of hydrogen and/or hydrogen gas, catalytic conversion (oxidation) of hydrogen to water or other compound.

The mechanism for catalytic conversion of hydrogen to reduce total hydrogen release to the body is summarized as follows:
1^{st} step: Hydrogen gas is produced by the degradation of Mg or Mg alloy.
2^{nd} step: Hydrogen is brought into contact with the catalytic site. The contact can be facilitated by one or more different processes, such as (but not limited to) adsorption and chemisorption. Hydrogen can be initially adsorbed by the catalytic site, or initially adsorbed by a site nearby the catalytic site, and then be brought into contact with the catalytic site following desorption.
3^{rd} step: Hydrogen is catalytically oxidized to water (or other compound) on the catalytic site.

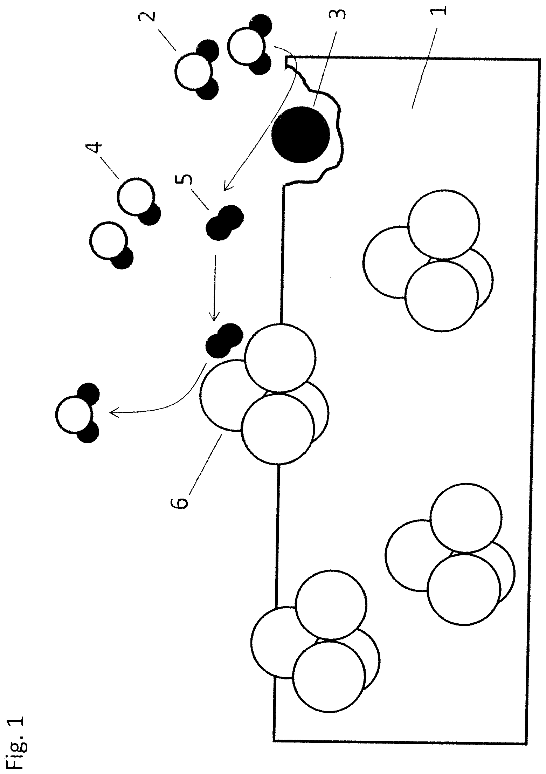

This process is illustrated by **Fig. 1**, where a matrix 1 is depicted, which matrix is made of magnesium or magnesium alloy. At the surface of the matrix 1, the electrochemical reaction as already mentioned above will occur in an aqueous solution (such as is found in tissue and/or blood and/or other fluid inside a patient):

Mg + 2H₂O → Mg²⁺ + 2OH⁻ + H₂

In the presence of water molecules 2 (small filled black circles depicting hydrogen atoms and unfilled larger circles depicting oxygen atoms), the magnesium in the matrix 1 will be degraded to magnesium ions 3 (depicted by a large filled black circle). Hydroxide ions 4 and gaseous hydrogen 5 are also formed in the reaction. In the presence of a catalyst 6 (depicted by large unfilled circles) which is dispersed within the matrix 1, the gaseous hydrogen 5 is catalytically converted to water molecules.

According to one aspect of the present disclosure, a composite material for an implant is provided, which composite material comprises:
- a matrix of magnesium or magnesium alloy, and
- a first catalyst which is dispersed within the matrix, wherein the first catalyst is adapted to catalytically convert the hydrogen gas to water or other compound; and
a second catalyst which is dispersed within the matrix, wherein the second catalyst is adapted to serve as a local and temporary storage sites for hydrogen gas, before the hydrogen gas is catalytically converted by the first catalyst.

The term "within the matrix" as used herein includes the situation where the catalyst is located on the surface of the matrix, the situation where the catalyst is located below the surface of the matrix, and also the situation where the catalyst is located both on the surface and also below the surface of the matrix. In one embodiment of the present disclosure, a catalyst system is incorporated in the metal bulk (magnesium or magnesium alloy) and/or on the surface of the metal bulk to promote hydrogen gas oxidation and potential following reactions of the reactant, wherein the reaction catalyst comprises platinum (Pt) or any one of the platinum group metals ruthenium (Ru), rhodium (Rh), palladium (Pd), osmium (Os), and iridium (Ir) or mixtures thereof. The catalyst, or mixes of catalysts, can be supported on another material, such as carbon black. Hereby, the hydrogen gas is catalytically converted to water or other compound, such as hydrogen ion or hydroxyl ion.

In another embodiment, the catalyst comprises metal oxides and/or carbides, such as vanadium (V) or nickel (Ni) based oxides, W based carbides or mixtures thereof.

In yet another embodiment, the catalyst comprises a compound that has high affinity for hydrogen adsorption and slow reaction kinetics for hydrogen evolution, such as niobium (Nb), molybdenum (Mo), tantalum (Ta) or tungsten (W) or mixtures thereof. These materials will serve as local and temporary storage sites for hydrogen. The hydrogen is thereafter desorbed from the adsorption site and catalytically converted at the catalytic site, which can be the same surface as the adsorption site or located elsewhere on the material. The hydrogen oxidation reaction is commonly catalysed by Platinum (Pt) or other platinum group metals (Pt, Ru, Pd, Ir and Rh) or mixes thereof. The catalyst can be supported on e.g. carbon black. Also Pt can be alloyed or mixed with other elements (including, but not limited to, e.g. Sn, Mo, Ni, Fe, Ag, W, Os, Co, Mn, and their oxides and carbides) to improve the catalytic properties, through various effects, such as bifunctional effects, ligand (electronic) effects and, ensemble (morphological) effects. Additional materials and techniques are described in Chapter 3.3 in, PEM Fuel Cell Electrocatalysts and Catalyst Layers: Fundamentals and Applications, (J. Zhang, author), 2008 Springer Verlag London Limited.

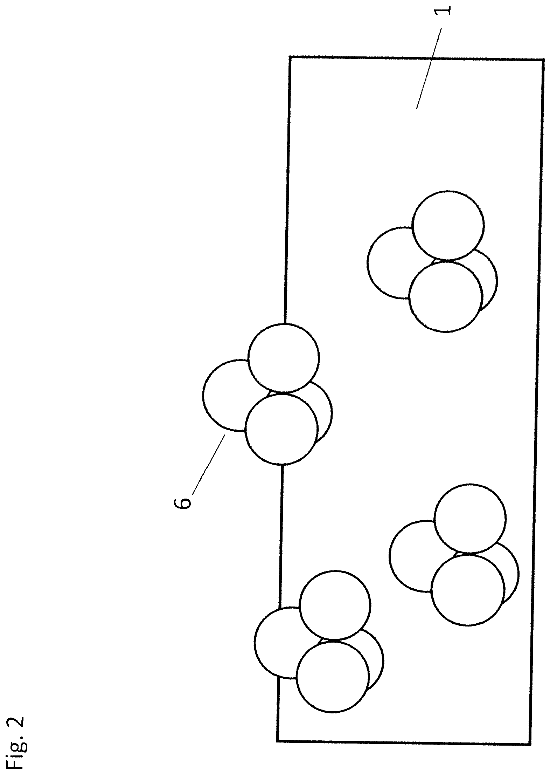

A composite material for an implant according to the present disclosure is illustrated in **Fig. 2**, depicting a matrix 1 made of magnesium or magnesium alloy, and a catalyst 6 dispersed throughout the matrix 1. The catalyst 6 comprises a catalytic compound or a mixture of different catalytic compounds, as described above, which may be dispersed within the matrix 1 in varying amounts. The catalyst 6 may be uniformly or non-uniformly distributed within the matrix 1. The amounts and distribution patterns of the catalyst are chosen such that there is always an amount of active catalyst present on the surface of the composite material.

The catalysts can be incorporated and dispersed into the material by several processes, such as mixing of catalytic powders into Mg (or Mg-alloy) powder followed by sintering into a material, or mixing of salt of the catalyst into Mg (or Mg-alloy) powder followed by sintering, or dispersion of solutions (aqueous or non-aqueous) containing the catalyst into the Mg (or Mg-alloy) powder followed by sintering. Alternatively, the catalysts may be incorporated into the Mg (or Mg-alloy) melt and if it is not soluble in the melt it will be dispersed into the melt by mixing and/or convection and maintain its structure upon cooling of the melt.

According to another aspect of the present disclosure, a medical implant is provided which comprises a composite material as disclosed above.

Such a medical implant makes it possible to achieve reduced hydrogen evolution while maintaining desirable mechanical and corrosion characteristics related to magnesium based materials. In addition, incorporation of catalysts into the magnesium based material allows for a means to control and tailor the degradation rate as different trace compounds affect the degradation time of magnesium. For example, trace levels of nickel, which is a powerful catalyst for hydrogen oxidation, at the same time greatly accelerates the degradation rate of magnesium.

Yet another aspect of the present disclosure relates to a method for incorporating a catalyst in the metal or metal alloy, including coating the surface of the metal or metal alloy with a catalyst, comprising sintering a mixture of the bulk or matrix material (Mg-based) and one or several catalysts. The sintering can be performed on powder mixtures by conventional sintering or by other forms of sintering, like spark plasma sintering, liquid phase sintering (addition of a material with a lower melting point than the matrix to be sintered, and electric current assisted sintering.

The catalyst (in powder form) can be directly mixed into the bulk powder material by mechanical means. An alternative method to deposit the catalyst is to first dissolve the catalyst in liquid (e.g. salt of Pt (e.g. H₂PtCl₆·6H₂O, or H₃Pt(SO₃)₂OH) in water, diluted acid or organic solvent), and then to deposit the catalyst, with or without the use of electrochemical assistance, onto the powder bulk material. The excess solvent is removed by evaporation, optionally assisted by heating. This is suitable when the catalyst material has a higher standard reduction potential (i.e. is more noble) than the (Mg) bulk material, as in the case of for example Pt, Pd, Ru, etc. (Bitter et al., Catalysis Today, 29 (1996) 349-353; Thompson et al., Journal of Electroanalytical Chemistry 515 (2001) 61-70).

Thus, the catalyst will be dispersed throughout the entire magnesium-based matrix. Consequently, there will always be active catalyst present on the surface, ready to take care of the hydrogen gas formed as the degradation of the magnesium-based material progresses. The catalyst will only become active when it appears on the surface of the material.

The implantable components can be directly produced by sintering into a desired shape or sintered into a basic shape and then reshaping and modifying into final shape, by for example grinding, rolling, or other metal shaping/processing method.

Specific applications of the present disclosure include the use of degradable coronary (or other blood contacting) stents in diabetic patients. In general, diabetic patients have a high risk of rapid restenosis (i.e. re-narrowing of blood vessels) and degradable stent technologies (see e.g. US2005010279A1) have been proposed to improve the outcome for these patients.

Another application of the present disclosure is extravascular closure devices in the form of metal clips for rapid and secure closure. The metal clip technique is advantageous as it has high mechanical stability, and is degradable and radio-opaque. The metal clip can, for example, be used to seal the hole in the vessel following a procedure requiring an arterial puncture. If the patient requires another arterial puncture before the clip is degraded it can easily be visualized by X-ray prior to the procedure, and the vessel can be punctured in another location to avoid the remaining clip being pushed into the vessel. X-ray visualisation is not possible with today's degradable closure devices as they are not X-ray visible. Another application of the present disclosure is vascular closure devices in the form of metal components for rapid and secure closure. The vascular closure device can comprise two degradable components (one or both made from degradable metal), one of which is located inside and one outside the blood vessel. The two components are then joined together (by for example a suture) to obtain haemostasis by mechanically blocking the hole in the vessel. Existing vascular devices use degradable polymers but degradable metal components are advantageous as they have more suitable mechanical properties, and are radio-opaque. Examples of such closure devices are described in detail in U.S. Patent Nos. 6,090,130; 6,508,828; 7,931,670; 7,250,057; 7,713,283; and 7,988,706.

The present disclosure also includes a composite material as described herein for use in a method for reducing the release of hydrogen gas from the matrix when the matrix is being degraded in an aqueous environment at a pH below 11.5, such as in the human or animal body.

Further, the present disclosure comprises a composite material or a medical implant as described herein for use in a method for reducing the release of hydrogen gas inside a patient, comprising inserting the composite material or medical implant inside the patient.

## Claims

1. A composite material for an implant, said composite material comprising:
a matrix of magnesium or magnesium alloy;
a first catalyst which is dispersed within the matrix, wherein the first catalyst is adapted to catalytically convert the hydrogen gas to water or other compound; and
a second catalyst which is dispersed within the matrix, wherein the second catalyst is adapted to serve as a local and temporary storage sites for hydrogen gas, before the hydrogen gas is catalytically converted by the first catalyst.

2. The composite material according to claim 1, wherein the matrix is adapted to being degraded in an aqueous environment at a pH below 11.5.

3. The composite material according to any one of claims 1-2, wherein the first and second catalysts have a concentration of 0.001 - 1% by weight of the composite material.

4. The composite material according to any one of claims 1-3, wherein the first and second catalysts are dispersed throughout the entire matrix of magnesium or magnesium alloy.

5. The composite material according to any one of claims 1-4, wherein the first and second catalysts are dispersed throughout the matrix such that there is always active catalyst present on a surface of the matrix.

6. The composite material according to claim 1, wherein the first catalyst comprises platinum (Pt) or other platinum group metals, or mixtures thereof, or a vanadium (V) based oxide, a nickel (Ni) based oxide, a W based carbide, or mixtures thereof; and
wherein the second catalyst comprises niobium (Nb), molybdenum (Mo), tantalum (Ta), tungsten (W), or mixtures thereof.

7. The composite material according to claim 6, wherein the first catalyst comprises platinum (Pt) or other platinum group metals selected from ruthenium (Ru), rhodium (Rh), palladium (Pd), osmium (Os), and iridium (Ir).

8. The composite material according to claim 6, wherein the first catalyst comprises platinum (Pt) or other platinum group metals selected from ruthenium (Ru), rhodium (Rh), palladium (Pd), and iridium (Ir).

9. The composite material according to claim 6, wherein the first catalyst is platinum (Pt) or wherein platinum is alloyed or mixed with other elements, including Sn, Mo, Ni, Fe, Ag, W, Os, Co, Mn, and their oxides and carbides.

10. A medical implant comprising a composite material according to any one of claims 1-9.

11. The medical implant according to claim 10, wherein the medical implant is selected from the group consisting of a coronary stent, a suture, a vascular closure device, a device for temporary attachment of a pacemaker or a neurostimulator, an orthopedic implant, an extravascular closure device, and a clip.

12. A method for preparing a composite material according to claim 1, comprising dispersing the first and second catalysts into the matrix by sintering a mixture of the matrix and the catalyst.

13. A method for producing a medical implant according to claim 10, comprising sintering the medical implant into a desired shape directly, or sintering the medical implant into a basic shape, followed by reshaping it into its final shape.

14. A method for producing a medical implant according to claim 13, wherein the reshaping includes at least one of grinding and rolling.

15. The composite material according to any one of claims 1-9 or the medical implant according to any one of claims 10-11 for use in a method for reducing release of hydrogen gas inside a patient.

## Patentansprüche

1. Verbundstoffmaterial für ein Implantat, wobei das Verbundstoffmaterial Folgendes umfasst:
eine Matrix aus Magnesium oder Magnesiumlegierung;
einen ersten Katalysator, der in der Matrix dispergiert ist, wobei der erste Katalysator so ausgelegt ist, dass er das Wasserstoffgas katalytisch zu Wasser oder einer anderen Verbindung umwandelt; und
einen zweiten Katalysator, der in der Matrix dispergiert ist, wobei der zweite Katalysator so ausgelegt ist, dass er als eine lokale und vorübergehende Speicherstelle für Wasserstoffgas dient, bevor das Wasserstoffgas durch den ersten Katalysator katalytisch umgewandelt wird.

2. Verbundstoffmaterial nach Anspruch 1, wobei die Matrix so ausgelegt ist, dass sie in einer wässrigen Umgebung bei einem pH-Wert unterhalb von 11,5 abgebaut wird.

3. Verbundstoffmaterial nach einem der Ansprüche 1-2, wobei der erste und der zweite Katalysator eine Konzentration von 0,001-1 Gew.-% des Verbundstoffmaterials aufweisen.

4. Verbundstoffmaterial nach einem der Ansprüche 1-3, wobei der erste und der zweite Katalysator in der gesamten Matrix aus Magnesium oder Magnesiumlegierung dispergiert sind.

5. Verbundstoffmaterial nach einem der Ansprüche 1-4, wobei der erste und der zweite Katalysator in der gesamten Matrix dispergiert sind, so dass an einer Oberfläche der Matrix immer ein aktiver Katalysator vorhanden ist.

6. Verbundstoffmaterial nach Anspruch 1, wobei der erste Katalysator Platin (Pt) oder andere Platingruppenmetalle oder Mischungen davon oder ein auf Vanadium (V) basierendes Oxid, ein auf Nickel (Ni) basierendes Oxid, ein auf W basierendes Carbid oder Mischungen davon umfasst; und
wobei der zweite Katalysator Niob (Nb), Molybdän (Mo), Tantal (Ta), Wolfram (W) oder Mischungen davon umfasst.

7. Verbundstoffmaterial nach Anspruch 6, wobei der erste Katalysator Platin (Pt) oder andere Platingruppenmetalle umfasst, die ausgewählt sind aus Ruthenium (Ru), Rhodium (Rh), Palladium (Pd), Osmium (Os) und Iridium (Ir).

8. Verbundstoffmaterial nach Anspruch 6, wobei der erste Katalysator Platin (Pt) oder andere Platingruppenmetalle umfasst, die ausgewählt sind aus Ruthenium (Ru), Rhodium (Rh), Palladium (Pd) und Iridium (Ir).

9. Verbundstoffmaterial nach Anspruch 6, wobei der erste Katalysator Platin (Pt) ist oder wobei Platin mit anderen Elementen legiert oder vermischt ist, einschließlich Sn, Mo, Ni, Fe, Ag, W, Os, Co, Mn und deren Oxide und Carbide.

10. Medizinisches Implantat umfassend ein Verbundstoffmaterial nach einem der Ansprüche 1-9.

11. Medizinisches Implantat nach Anspruch 10, wobei das medizinische Implantat ausgewählt ist aus der Gruppe bestehend aus einem koronaren Stent, einem Nahtmaterial, einer Gefäßverschlussvorrichtung, einer Vorrichtung zum vorübergehenden Befestigen eines Schrittmachers oder eines Neurostimulators, einem orthopädischen Implantat, einer extravasalen Verschlussvorrichtung und einer Klammer.

12. Verfahren zum Herstellen eines Verbundstoffmaterials nach Anspruch 1, umfassend das Dispergieren des ersten und zweiten Katalysators in die Matrix durch Sintern einer Mischung der Matrix and des Katalysators.

13. Verfahren zum Erzeugen eines medizinischen Implantats nach Anspruch 10, umfassend das Sintern des medizinischen Implantats direkt in eine gewünschte Form oder das Sintern des medizinischen Implantats in eine Grundform, gefolgt vom Umformen dieser in ihre endgültige Form.

14. Verfahren zum Erzeugen eines medizinischen Implantats nach Anspruch 13, wobei das Umformen wenigstens eines aus Schleifen und Walzen beinhaltet.

15. Verbundstoffmaterial nach einem der Ansprüche 1-9 oder das medizinische Implantat nach einem der Ansprüche 10-11 für die Verwendung in einem Verfahren zum Verringern der Wasserstoffgasfreisetzung im Inneren eines Patienten.

## Revendications

1. Matériau composite pour un implant, ledit matériau composite comprenant :
une matrice de magnésium ou d'alliage de magnésium ;
un premier catalyseur qui est dispersé à l'intérieur de la matrice, dans lequel le premier catalyseur est conçu pour convertir catalytiquement l'hydrogène gazeux en eau ou en un autre composé ; et
un second catalyseur qui est dispersé à l'intérieur de la matrice, dans lequel le second catalyseur est conçu pour servir de site de stockage local et temporaire pour l'hydrogène gazeux, avant la conversion catalytique de l'hydrogène gazeux par le premier catalyseur.

2. Matériau composite selon la revendication 1, dans lequel la matrice est conçue pour être dégradée dans un environnement aqueux à un pH inférieur à 11,5.

3. Matériau composite selon l'une quelconque des revendications 1 à 2, dans lequel le premier et le second catalyseurs ont une concentration de 0,001 à 1 % en poids du matériau composite.

4. Matériau composite selon l'une quelconque des revendications 1 à 3, dans lequel le premier et le second catalyseurs sont dispersés à travers la totalité de la matrice de magnésium ou d'alliage de magnésium.

5. Matériau composite selon l'une quelconque des revendications 1 à 4, dans lequel le premier et le second catalyseurs sont dispersés à travers la matrice de sorte qu'il y ait toujours un catalyseur actif présent sur une surface de la matrice.

6. Matériau composite selon la revendication 1, dans lequel le premier catalyseur comprend du platine (Pt) ou d'autres métaux du groupe du platine, ou des mélanges de ceux-ci, ou un oxyde à base de vanadium (V), un oxyde à base de nickel (Ni), un carbure à base de W, ou des mélanges de ceux-ci ; et
dans lequel le second catalyseur comprend du niobium (Nb), du molybdène (Mo), du tantale (Ta), du tungstène (W), ou des mélanges de ceux-ci.

7. Matériau composite selon la revendication 6, dans lequel le premier catalyseur comprend du platine (Pt) ou d'autres métaux du groupe du platine choisis parmi le ruthénium (Ru), le rhodium (Rh), le palladium (Pd), l'osmium (Os) et l'iridium (Ir).

8. Matériau composite selon la revendication 6, dans lequel le premier catalyseur comprend du platine (Pt) ou d'autres métaux du groupe du platine choisis parmi le ruthénium (Ru), le rhodium (Rh), le palladium (Pd) et l'iridium (Ir).

9. Matériau composite selon la revendication 6, dans lequel le premier catalyseur est du platine (Pt) ou dans lequel le platine est allié ou mélangé avec d'autres éléments, comprenant le Sn, Mo, Ni, Fe, Ag, W, Os, Co, Mn, et leurs oxydes et carbures.

10. Implant médical comprenant un matériau composite selon l'une quelconque des revendications 1 à 9.

11. Implant médical selon la revendication 10, dans lequel l'implant médical est choisi dans le groupe composé d'un stent coronaire, d'une suture, d'un dispositif de fermeture vasculaire, d'un dispositif pour la fixation temporaire d'un stimulateur cardiaque ou d'un neurostimulateur, d'un implant orthopédique, d'un dispositif de fermeture extravasculaire et d'une pince.

12. Procédé de préparation d'un matériau composite selon la revendication 1, comprenant la dispersion des premier et second catalyseurs dans la matrice en frittant un mélange de la matrice et du catalyseur.

13. Procédé de production d'un implant médical selon la revendication 10, comprenant le frittage de l'implant médical directement en une forme souhaitée, ou le frittage de l'implant médical en une forme de base, suivi d'un remodelage pour lui donner sa forme finale.

14. Procédé de production d'un implant médical selon la revendication 13, dans lequel le remodelage comprend au moins l'un du meulage et du laminage.

15. Matériau composite selon l'une quelconque des revendications 1 à 9 ou implant médical selon l'une quelconque des revendications 10 à 11, pour une utilisation dans un procédé permettant de réduire la libération d'hydrogène gazeux à l'intérieur d'un patient.
